# FASCICULE DE BREVET EUROPEEN

(11) **EP 2 814 598 B1**
(45) Date de publication et mention de la délivrance du brevet: **09.05.2018**
(21) Numéro de dépôt: 13704424.4
(22) Date de dépôt: 13.02.2013
(51) Int. Cl.: B01J 13/14

(54) **PROCÉDÉ DE FONCTIONNALISATION DE NANO-OBJETS EN CARBONE**
VERFAHREN ZUR FUNKTIONALISIERUNG VON KOHLENSTOFFNANOOBJEKTEN
METHOD FOR FUNCTIONALISING CARBON NANO-OBJECTS

(30) Priorité: 13.02.2012 FR 1251331
(43) Date de publication de la demande: 24.12.2014
(73) Titulaire: Commissariat à l'Énergie Atomique et aux Énergies Alternatives, 75015 Paris (FR)
(72) Inventeur: CAMPIDELLI, Stéphane, 78120 Rambouillet (FR); CLAVE, Guillaume, 78990 Elancourt (FR)
(74) Mandataire: Brevalex
(86) Numéro de dépôt international: PCT/EP2013/052892
(87) Numéro de publication internationale: WO 2013/120907

(56) Documents cités:
- US-A1- 2011 076 497

## Description

### DOMAINE TECHNIQUE

L'invention se rapporte au domaine de la fonctionnalisation de nano-objets en carbone.

Elle se rapporte plus spécifiquement à un procédé permettant de fonctionnaliser des nano-objets en carbone et, en particulier, des nanotubes de carbone et des nanofeuillets de graphène.

Sont également décrites une composition comprenant des nano-objets fonctionnalisés par ce procédé, en suspension dans un solvant organique, ainsi que les utilisations de cette composition.

La fonctionnalisation de nano-objets en carbone telle que réalisée selon l'invention permet, en premier lieu, de conférer à ces nano-objets une aptitude à être dispersés et, donc, à être individualisés dans les solvants organiques - ce qui facilite grandement leur manipulation et leur utilisation - et ce sans altérer la structure de ces nano-objets et, par voie de conséquence, les propriétés, notamment électriques et optiques, qu'ils présentent naturellement.

De ce fait, l'invention est susceptible d'être utilisée, d'une manière générale, dans tous les domaines dans lesquels les nano-objets en carbone présentent potentiellement des applications.

Cette fonctionnalisation permet, si on le souhaite, de conférer de plus à des nano-objets en carbone des propriétés comme, par exemple, des propriétés de compatibilité vis-à-vis des résines polymères classiquement utilisées dans la fabrication de composites (résines époxydes, résines vinylesters, ...), des propriétés d'absorption de photons et de transfert de ces photons et/ou de charges photoinduites, des propriétés d'oxydoréduction, des propriétés de catalyse, des propriétés de reconnaissance et de ciblage de molécules chimiques ou biologiques, des propriétés de vectorisation de molécules chimiques ou biologiques, des propriétés de magnétisme, et d'autres encore.

L'invention est donc susceptible d'être utilisée plus particulièrement dans l'élaboration :
- de matériaux composites et, en particulier, de matériaux nanocomposites ;
- de matériaux destinés à collecter l'énergie de la lumière et à la convertir en électricité (photovoltaïque) ;
- de dispositifs de détection du type capteurs/senseurs ou biocapteurs/biosenseurs ;
- de systèmes de catalyse ou de photocatalyse ;
- de systèmes de vectorisation ciblée de composés d'intérêt thérapeutique (médicaments, vaccins, etc) ou diagnostique ; et
- d'agents de contraste pour l'imagerie médicale, par exemple pour l'imagerie par résonance magnétique (IRM).

### ÉTAT DE LA TECHNIQUE ANTÉRIEURE

Les nano-objets en carbone et, en particulier, les nanotubes de carbone (ou NTCs) et les nanofeuillets de graphène, suscitent un énorme intérêt dans le monde de la recherche, tant appliquée que fondamentale, en raison de leurs propriétés qui sont exceptionnelles à bien des égards.

Ils présentent toutefois l'inconvénient d'être très difficiles à disperser et à individualiser dans les solvants organiques classiquement utilisés dans l'industrie chimique, ce qui constitue un réel frein à leur utilisation.

Pour résoudre ce problème, il a été proposé de fonctionnaliser les NTCs et le graphène.

Deux grandes voies de fonctionnalisation ont été explorées, à savoir :
- une fonctionnalisation dite « *covalente* », qui consiste à fixer des groupes chimiques hydrophobes par des liaisons covalentes sur la surface des NTCs ou des nanofeuillets de graphène ; et
- une fonctionnalisation dite « *non-covalente* », qui consiste à adsorber des composés hydrophobes *via* des interactions non covalentes du type « π-*stacking* », interactions de van-der-Waals ou autres, à la surface des NTCs ou des nanofeuillets de graphène.

Chacune de ces voies est efficace en soi mais a malheureusement ses inconvénients.

Ainsi, la fonctionnalisation « *covalente* » présente l'inconvénient de détruire partiellement le réseau de carbone sp² des NTCs et des nanofeuillets de graphène (chaque site de greffage transforme un carbone sp² en carbone sp³) et, par là-même, d'en altérer les propriétés électriques et optiques, tandis que la fonctionnalisation *« non covalente* » présente l'inconvénient que les interactions établies entre les composés adsorbés et les NTCs ou les nanofeuillets de graphène sont très fragiles et sont donc susceptibles d'être détruites lors de la manipulation ultérieure des NTCs ou des nanofeuillets de graphène, par exemple par une filtration ou un chauffage.

C'est ce qui a amené K. J. Ziegler et *al.* à proposer, dans la demande de brevet US 2011/0076497, ci-après référence **[1],** un procédé de fonctionnalisation « *non covalente* » dans lequel un polymère est formé autour de NTCs par polymérisation *in situ.*

Ce procédé consiste à disperser des NTCs dans une solution aqueuse d'un agent tensio-actif pour obtenir une suspension aqueuse de NTCs, à mélanger cette suspension avec une solution contenant un premier monomère insoluble dans l'eau dans un solvant organique de sorte à obtenir un nano-environnement de type émulsion autour des NTCs, à introduire un deuxième monomère qui, lui, est soluble dans l'eau, dans le nano-environnement de sorte que les premier et deuxième monomères réagissent ensemble à l'interface entre les parties aqueuse et organique dudit nano-environnement pour former un polymère, et à éliminer éventuellement le solvant organique, l'eau et l'agent tensio-actif par lyophilisation, filtration, lavage et extraction.

Bien qu'intéressant, le procédé de fonctionnalisation « *non covalente »* décrit dans la référence **[1]** présente un certain nombre d'inconvénients et de limites.

En effet, ce procédé conduit à des NTCs qui sont fonctionnalisés par un polymère qui ne se trouve pas au plus près de la surface de ces NTCs et qui renferme des molécules de tensioactif que l'on ne peut pas éliminer, ce qui peut avoir un rôle négatif lors de l'exploitation des propriétés conférées aux NTCs par ce polymère.

Par ailleurs, bien qu'il soit dit, dans la référence **[1]**, que le polymère peut être un homopolymère, le procédé décrit dans cette référence ne permet en réalité de fonctionnaliser des NTCs que par des copolymères puisqu'il met en oeuvre deux monomères qui sont nécessairement différents, l'un étant soluble dans l'eau tandis que l'autre ne l'est pas.

D'ailleurs, le seul exemple concret d'application de ce procédé que comporte la référence **[1]** concerne la fonctionnalisation de NTCs par un copolymère de condensation, en l'espèce du nylon 6,10, qui est obtenu par réaction entre du chlorure de sébacoyle (qui est utilisé en solution dans du tétrachlorure de carbone) et de l'hexaméthylène diamine (qui est utilisée en solution aqueuse).

Selon la référence **[1],** le polymère qui sert à fonctionnaliser les NTCs peut être réticulé. Toutefois, si l'on prend le cas du seul exemple concret de fonctionnalisation qui est décrit dans cette référence, à savoir la fonctionnalisation par du nylon, il s'agit d'une fonctionnalisation par un polymère non réticulé, dont les chaînes polymères interagissent faiblement entre elles et de manière non covalente, ce qui rend fragile cette fonctionnalisation.

En outre, la fonctionnalisation de NTCs par du nylon est basée sur la réaction entre un groupe chlorure d'acide et un groupe amine. Or, ce type de réaction s'accompagne d'une production d'acide chlorhydrique dans le milieu réactionnel et donc, d'une acidification de ce milieu, ce qui peut provoquer une dégradation des molécules en solution, une déstabilisation du milieu réactionnel et une précipitation des nanotubes de carbone.

Il est de plus connu que les chlorures d'acide réagissent avec l'eau pour donner des acides carboxyliques qui ne sont pas capables de réagir avec des amines pour former des amides. La réactivité du groupe chlorure d'acide vis-à-vis de l'eau constitue donc une limite supplémentaire à l'utilisation de ce groupe en tant que groupe polymérisable.

Enfin, la fonctionnalisation de NTCs par des molécules de nylon n'est pas réversible en ce sens qu'il est impossible de débarrasser les NTCs du nylon qui les entoure et de recycler ces NTCs si on le souhaite.

Un procédé de fonctionnalisation par du nylon, analogue à celui décrit dans la référence **[1]**, a également été proposé pour des nanofeuillets de graphène par Sriya Das et al. dans ACS Applied Materials & Interfaces 2011, 3, 1844-1851, ci-après référence **[2].** Les inconvénients et limites de ce procédé sont les mêmes que ceux précédemment mentionnés pour la fonctionnalisation de NTCs décrite dans la référence **[1].**

Les Inventeurs se sont donc fixé pour but de fournir un procédé de fonctionnalisation « *non covalente* » de nano-objets en carbone et, plus spécifiquement, de NTCs et de nanofeuillets de graphène, qui soit exempt des multiples inconvénients présentés par les procédés de fonctionnalisation « *non covalente* » décrits à ce jour et, en particulier, par les procédés proposés dans les références **[1]** et **[2]** précitées.

En particulier, les Inventeurs se sont fixé pour but de fournir un procédé de fonctionnalisation « *non covalente* » de nano-objets en carbone qui permette de créer véritablement un réseau formé d'un polymère réticulé autour de ces nano-objets et ce, sans que des molécules de tensioactif ne soient emprisonnées dans ce réseau, et qui permette de choisir ce polymère dans une très grande gamme de possibilités (homo- et copolymères, polymères organiques et organo-inorganiques, etc).

### EXPOSÉ DE L'INVENTION

Ces buts et d'autres encore sont atteints par l'invention qui propose, en premier lieu, un procédé de fonctionnalisation de nano-objets en carbone par formation d'au moins une couche d'un polymère réticulé autour de ces nano-objets, lequel procédé comprend au moins les étapes suivantes :
a) la dispersion des nano-objets dans une solution aqueuse d'un tensioactif pour obtenir une suspension dans laquelle chaque nano-objet est entouré de molécules de tensioactif, la partie hydrophobe de ces molécules étant orientée vers le nano-objet et la partie hydrophile desdites molécules étant en contact avec l'eau de la suspension ;
b) le mélange de la suspension ainsi obtenue avec une solution comprenant un ou plusieurs monomères organiques et/ou organo-inorganiques dans un solvant organique non miscible à l'eau, le ou les monomères ayant un coefficient de partage dichloro-méthane/eau déionisée au moins égal à 5 à une température de 25°C et comportant au moins trois groupes chimiques polymérisables choisis parmi les groupes thiols et sélénols, éventuellement protégés par un groupe protecteur, ce mélange étant réalisé sous agitation , moyennant quoi la solution de monomère(s) est amenée à l'interface entre les nano-objets et les molécules de tensioactif qui entourent ces nano-objets ;
c) l'élimination du solvant organique du mélange ;
d) la polymérisation et la réticulation du ou des monomères à l'interface entre les nano-objets et les molécules de tensioactif qui entourent ces nano-objets, après déprotection des groupes chimiques polymérisables si ceux-ci sont protégés, pour obtenir la formation d'une couche de polymère réticulé autour des nano-objets, cette couche étant elle-même entourée par les molécules de tensioactif ;
e) l'élimination des molécules de tensio-actif qui entourent la couche de polymère réticulé ; et
f) la récupération des nano-objets ainsi fonctionnalisés.

Ainsi, selon l'invention, non seulement on utilise un ou plusieurs monomères qui comportent chacun au moins trois groupes chimiques polymérisables, ce qui permet de former un réseau formé d'un polymère réticulé autour des nano-objets que l'on souhaite fonctionnaliser, mais, de plus, la réaction de polymérisation/réticulation qui conduit à la formation de ce réseau est réalisée à l'interface entre les nano-objets et les molécules de tensioactif qui entourent ces nano-objets (et non pas à l'interface entre les molécules de tensioactif et l'eau de la suspension comme décrit dans les références **[1]** et **[2]** précitées). Le réseau polymère se forme donc au plus près de la surface des nano-objets et ce, sans emprisonner de molécules de tensioactif.

Il en résulte que le procédé de l'invention conduit à des nano-objets fonctionnalisés qui sont extrêmement stables et résistants, qui peuvent être aisément manipulés et, notamment, être purifiés sans risque d'altération ni de perte de matière.

Dans ce qui précède et ce qui suit, on entend par « *nano-objets en carbone* », des objets constitués de carbone et qui présentent, conformément à la norme ISO TS 80004-3 : 2010, une, deux ou trois dimensions externes à l'échelle nanométrique, c'est-à-dire allant de 1 à 100 nm. Dans le cadre de l'invention, il s'agit typiquement de nanotubes de carbone mono- ou multiparois, ou de nanofeuillets mono- ou multicouches. Il peut, toutefois, également s'agir de nanofibres de carbone ou de nano-oignons de carbone.

Par ailleurs, on entend par « *polymère* », aussi bien un homopolymère qui est issu d'un seul monomère et qui est donc formé d'un seul motif répétitif, qu'un copolymère qui est issu de plusieurs (c'est-à-dire, deux ou plus de deux) monomères différents et qui est donc formé de plusieurs motifs répétitifs différents.

C'est la raison pour laquelle la solution organique utilisée à l'étape b) du procédé de l'invention peut comprendre un seul monomère ou, au contraire, une pluralité de monomères différents, selon que l'on veut fonctionnaliser les nano-objets par au moins une couche d'un homopolymère réticulé ou par au moins une couche d'un copolymère réticulé.

Le ou les monomères susceptibles d'être utilisés dans le cadre de l'invention peuvent être choisis parmi de très nombreux composés organiques ou organo-inorganiques pour autant que ces composés satisfassent aux deux conditions suivantes :
(1) avoir un coefficient de partage dichlorométhane/eau au moins égal à 5 à une température de 25°C, pour pouvoir conférer aux nano-objets une aptitude à être dispersés et individualisés dans les solvants organiques ; et
(2) comporter au moins trois groupes chimiques polymérisables choisis parmi les groupes thiols et sélénols, pour permettre la formation d'un réseau polymère réticulé autour de ces nano-objets.

On rappelle que le coefficient de partage, généralement noté K, d'un composé entre deux solvants non miscibles est égal au rapport des concentrations molaires que présente ce composé dans ces solvants après avoir été ajouté à un milieu comprenant les deux solvants en contact l'un avec l'autre et laissé libre de se répartir entre chacun d'eux, ces opérations étant réalisées à une température θ donnée.

Dans le cadre de l'invention, les solvants à prendre en considération pour déterminer le coefficient de partage d'un composé sont le dichloro-méthane et l'eau déionisée en sorte que ce coefficient de partage correspond au rapport entre la concentration molaire que présente ce composé dans le dichlorométhane et la concentration molaire que présente ce même composé dans l'eau déionisée, tandis que la température θ à prendre en considération est de 25°C.

Le coefficient de partage dichlorométhane/ eau déionisée d'un composé peut notamment être déterminé par la méthode dite « *du flacon agité* » ou « *par agitation en flacon* », qui reste à ce jour l'une des méthodes les plus fiables pour déterminer le coefficient de partage d'un composé entre deux solvants non miscibles. Cette méthode consiste à ajouter, dans un flacon, une quantité connue du composé à un milieu formé par les deux solvants à parts égales, à agiter le flacon, à laisser décanter les deux phases en présence. On mesure ensuite la concentration du composé dans chacune des phases, par exemple par spectroscopie UV-visible. Les mesures sont, de préférence, faites 3 fois et le coefficient de partage est déterminé en calculant la moyenne de ces mesures.

Des composés susceptibles d'être utilisés comme monomères dans le procédé de l'invention peuvent notamment être des composés dotés d'un chromophore, c'est-à-dire d'un groupe d'atomes qui comporte une ou plusieurs doubles liaisons et qui forme avec le reste de la molécule une alternance de doubles et simples liaisons. De tels composés peuvent, en effet, être utilisés pour conférer aux nano-objets des propriétés optiques particulières, par exemple d'absorption de photons et de transfert de ces photons et/ou de charges photo-induites.

Ainsi, il peut notamment s'agir de composés qui comprennent un groupe azobenzène, anthraquinone, indigotine, triarylméthane tel que triphénylméthane, acridine, xanthène, β-carotène, quinoline, chlorine, porphyrine, phtalocyanine, naphtalocyanine, fluorescéine, rhodamine, BODIPY (bore-dipyrométhène), coumarine ou cyanine (par exemple, du type cyanine 3, cyanine 5 ou cyanine 7), convenablement substitué pour comporter au moins trois groupes chimiques polymérisable.

Lorsqu'il s'agit d'un groupe chlorine, porphyrine, phtalocyanine ou naphtalocyanine, ce groupe peut être sous forme base libre, auquel cas il est dépourvu de tout atome de métal ou, au contraire, être métallé, c'est-à-dire coordonné à un cation métallique, typiquement d'un atome de métal de transition tel que le zinc, le cuivre, le nickel, le cobalt, le fer, l'or ou le platine.

Des composés susceptibles d'être utilisés comme monomères dans le procédé de l'invention peuvent également être des complexes d'un métal de transition, dans lesquels ce métal est coordonné à plusieurs molécules d'un même ligand organique (le complexe est alors dit homoleptique) ou à plusieurs molécules correspondant à des ligands organiques différents (le complexe est alors dit hétéroleptique). Selon le métal de transition qu'ils comprennent, de tels complexes peuvent, en effet, être utilisés pour conférer aux nano-objets des propriétés de catalyse, de photocatalyse, de magnétisme, etc, utiles notamment pour des applications en électrochimie.

Ainsi, par exemple, il peut s'agir de complexes d'hexapyridine, de tris(2,2'-bipyridine) ou de bis(2,2':6',2'-terpyridine) de fer, de cobalt ou de ruthénium, dont on a convenablement substitué les molécules de pyridine, de 2,2'-bipyridine ou de 2,2':6',2'-terpyridine de sorte que ces complexes puissent comporter au moins trois groupes chimiques polymérisables.

Des composés susceptibles d'être utilisés comme monomères dans le procédé de l'invention peuvent aussi être des complexes d'une terre rare (ou lanthanide), dans laquelle la terre rare est coordonnée à plusieurs molécules d'un même ligand organique (le complexe est alors dit homoleptique) ou à plusieurs molécules correspondant à des ligands organiques différents (le complexe est alors dit hétéroleptique), le ou les ligands pouvant notamment être des composés comprenant un groupe porphyrine ou phtalocyanine.

Selon la terre rare qu'ils comprennent, de tels complexes peuvent être utilisés pour conférer aux nano-objets des propriétés magnétiques (par exemple, si la terre rare est le terbium ou l'holmium) ou des propriétés de luminescence (par exemple, si la terre rare est l'europium).

Des composés susceptibles d'être utilisés comme monomères dans le procédé de l'invention peuvent encore consister en des nanoparticules inorganiques stabilisées par un ligand organique.

Ces nanoparticules peuvent notamment être des nanoparticules d'un métal tel que l'or, d'un alliage métallique, d'un semi-conducteur tel que le germanium ou le tellure, d'un alliage à propriétés semi-conductrices tel que le séléniure de cadmium CdSe ou le tellurure de cadmium CdTe, ou d'un oxyde métallique tel que l'oxyde de titane TiO₂ ou l'oxyde de fer(II,III), selon les propriétés (photocatalyse, magnétisme, etc) que l'on souhaite conférer aux nano-objets.

Dans le cadre de l'invention, les groupes chimiques polymérisables sont des groupes thiols ou des groupes sélénols en raison de ce que, lors de la polymérisation/réticulation du ou des monomères, ces groupes conduisent, pour les premiers, à la formation de liaisons -S-S- et, pour les seconds, à la formation de liaisons -Se-Se-, qu'il est possible de détruire ultérieurement au moyen d'un agent réducteur inorganique du type hydrure (borohydrure de sodium, aluminohydrure de lithium, hydrure de diisobutyl-aluminium ou analogue) ou par
réaction avec un excès d'un composé thiolé (2-mercaptoéthanol, dithiotréitol ou analogue) en milieu aqueux légèrement alcalin.

Ainsi, l'utilisation de groupes thiols ou sélénols en tant que groupes chimiques polymérisables, offre la possibilité de débarrasser ultérieurement les nano-objets ayant été fonctionnalisés par le procédé de l'invention de la couche de polymère qui les entoure et de recycler ces nano-objets si on le souhaite.

Par ailleurs, on préfère que le ou les monomères comportent au moins quatre groupes chimiques polymérisables et que les groupes chimiques polymérisables soient situés à l'extrémité de groupes espaceurs que comportent le ou les monomères, notamment pour éviter des problèmes d'encombrement stérique. Ces groupes espaceurs sont avantageusement des groupes hydrocarbonés linéaires, typiquement saturés, qui comprennent de 2 à 8 atomes de carbone et éventuellement un ou plusieurs hétéroatomes.

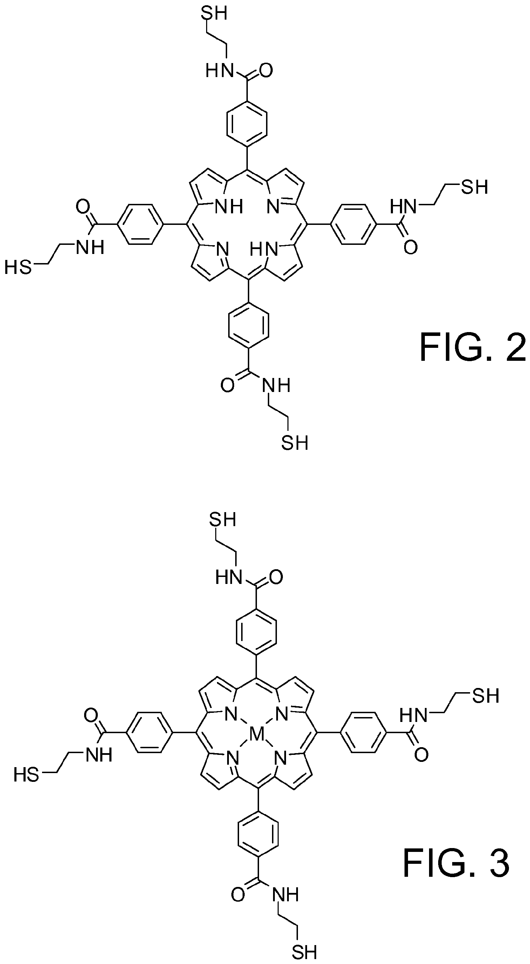

Des exemples de monomères organiques et organométalliques convenant particulièrement bien à la mise en oeuvre du procédé de l'invention sont illustrés sur les figures 2, 3, 10 et 12 annexées.

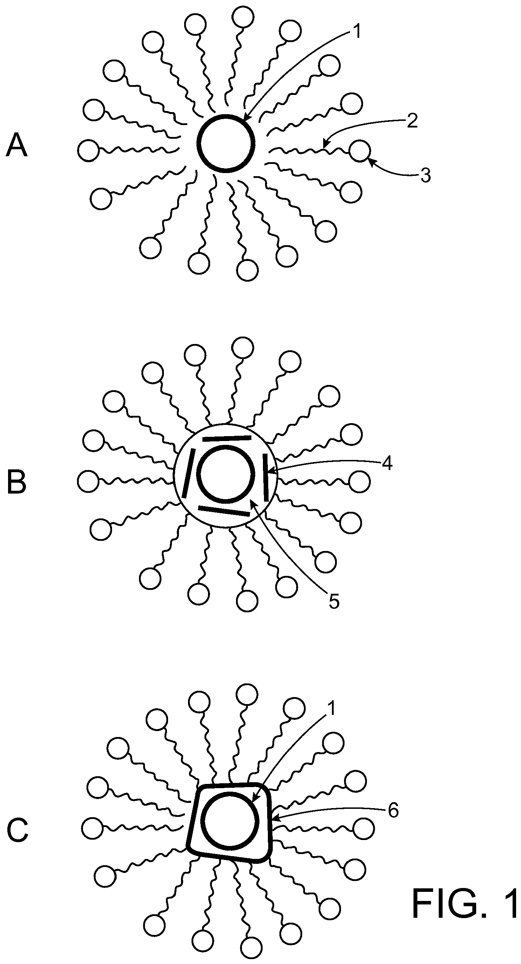

Comme précédemment mentionné, la première étape du procédé de l'invention, ou étape a), consiste à disperser les nano-objets dans une solution aqueuse d'un tensioactif pour obtenir une suspension dans laquelle chaque nano-objet est entouré de molécules de tensioactif, la partie hydrophobe des molécules de tensioactif étant dirigée vers le nano-objet et la partie hydrophile de ces molécules étant en contact avec l'eau de la suspension, comme schématiquement illustré sur la partie A de la figure 1 annexée.

Sur cette figure, la référence 1 correspond à un nano-objet, vu en coupe transversale, qui a été arbitrairement choisi à section droite circulaire mais que l'on aurait pu représenter avec une section droite différente, par exemple rectangulaire ; la référence 2 correspond à la partie hydrophobe des molécules de tensioactif entourant le nano-objet tandis que la référence 3 correspond à la partie hydrophile des molécules de tensioactif.

Conformément à l'invention, le tensioactif peut être tout tensioactif connu pour permettre la dispersion de nano-objets en carbone dans une solution aqueuse. Ainsi, il peut notamment s'agir du cholate de sodium, du dodécylsulfate de sodium, du 4-dodécyl-benzènesulfonate de sodium, du bromure de triméthylcétylammonium, d'un polysorbate tel que le Tween™ 20, ou encore du tensioactif qui est commercialisé sous la dénomination Triton X-100™.

La dispersion des nano-objets peut notamment être réalisée en ajoutant ces nano-objets à une solution aqueuse comprenant le tensioactif à une concentration au moins égale à la concentration micellaire critique (CMC) de ce tensioactif et, de préférence, au moins égale à 1,2 fois cette CMC, et en soumettant l'ensemble à une sonication. Si besoin est, la suspension ainsi obtenue peut être centrifugée pour en éliminer les nano-objets susceptibles d'être restés, malgré la sonication, sous forme d'agrégats.

L'étape suivante, ou étape b), comprend le mélange de la suspension de nano-objets avec une solution comprenant le ou les monomères dans un solvant organique non miscible à l'eau tel que le dichlorométhane, le chloroforme ou le toluène, et ce, sous agitation pour permettre à la solution de monomère(s) de pénétrer entre les molécules de tensioactif qui entourent les nano-objets et d'atteindre l'interface entre ces nano-objets et ces molécules, comme schématiquement illustré sur la partie B de la figure 1 annexée.

Sur cette figure, la référence 4 correspond à des molécules de monomère(s) tandis que la référence 5 correspond au solvant organique.

Le mélange de la suspension de nano-objets avec la solution de monomère(s) peut être réalisé en ajoutant cette suspension à cette solution (ou l'inverse) et en soumettant l'ensemble à une sonication.

Il est à noter qu'à ce stade, le ou les monomères peuvent être présents dans le solvant organique sous une forme dans laquelle leurs groupes chimiques polymérisables sont protégés par un groupe protecteur, si ceux-ci ont tendance à spontanément réagir, notamment en présence d'oxygène.

Ainsi, chacun des groupes thiols ou sélénols est avantageusement protégé, par exemple par un groupe acétyle, benzoyle ou analogue.

L'étape suivante, ou étape c), comprend l'élimination du solvant organique du mélange, ce qui peut notamment être réalisé en élevant la température de ce mélange tout le soumettant à une sonication.

Dans ce cas, la température à laquelle est porté le mélange doit être suffisante pour permettre l'évaporation du solvant organique mais doit néanmoins être inférieure à la température à partir de laquelle l'arrangement des molécules de tensioactif autour des nano-objets risque d'être détruit.

Pour ce faire, il est souhaitable que le mélange soit porté à une température qui ne dépasse pas 50°C, typiquement de 40 à 50°C. Si cette température est insuffisante pour évaporer le solvant organique compte-tenu de la température d'ébullition de ce solvant (ce qui sera, par exemple, le cas si le solvant organique est du toluène), alors l'étape c) est avantageusement réalisée en combinant l'élévation de température à une mise sous vide du mélange.

L'étape suivante, ou étape d), comprend la polymérisation et la réticulation du ou des monomères à l'interface entre les nano-objets et les molécules de tensioactif qui entourent ces nano-objets, pour obtenir la formation d'une couche d'un polymère réticulé autour des nano-objets, cette couche étant elle-même entourée par les molécules de tensioactif, comme schématiquement illustré sur la partie C de la figure 1 annexée.

Sur cette figure, la référence 6 correspond à la couche de polymère réticulé formée autour du nano-objet.

Les conditions dans lesquelles la polymérisation et la réticulation sont réalisées dépendent de la nature des groupes chimiques polymérisables que comportent le ou les monomères, étant entendu qu'il est souhaitable que ces opérations soient effectuées à une température qui ne dépasse pas 50°C pour éviter, là également, que l'arrangement des molécules de tensioactif autour des nano-objets ne soit détruit.

Dans le cas où les groupes chimiques polymérisables du ou des monomères sont protégés par un groupe protecteur, la polymérisation et la réticulation du ou des monomères sont précédées par une déprotection des groupes chimiques polymérisables.

Ainsi, par exemple, dans le cas où les groupes thiols ou sélénols sont protégés par un groupe acétyle, l'étape d) comprend avantageusement le traitement des nano-objets obtenus à l'issue de l'étape c) :
- par un agent de désacétylation du type hydroxylamine, hydrazine ou ammoniac, que l'on utilise en excès par rapport aux groupes thiols ou sélénols, pour déprotéger ces groupes ; puis
- par une base du type triéthylamine ou diisopropyléthylamine, que l'on utilise également en excès par rapport aux groupes thiols ou sélénols, sous une atmosphère oxydante, par exemple, sous un flux d'oxygène, pour induire la polymérisation et la réticulation du ou des monomères par réaction de ces groupes entre eux.

Ce traitement est, de préférence, réalisé à température ambiante.

Si besoin est, il va de soi que des composés de nature à initier et/ou à accélérer la polymérisation et la réticulation du ou des monomères peuvent être utilisés à l'étape d).

L'étape suivante, ou étape e), comprend l'élimination des molécules de tensioactif qui entourent la couche de polymère réticulé.

Cette opération peut notamment être réalisée en filtrant la suspension de nano-objets obtenue à l'issue de l'étape d) sur une membrane résistante aux solvants organiques et dont la porosité permet de retenir uniquement les nano-objets, par exemple une membrane en polytétrafluoroéthylène (Teflon™) de porosité de 0,2 µm, et en rinçant successivement les nano-objets avec de larges volumes d'eau et de différents solvants organiques tels que le méthanol, l'acétone, le tétrahydrofurane, la *N-*méthylpyrrolidone, le dichlorométhane et le diéthyl-éther.

Les nano-objets fonctionnalisés, ainsi débarrassés des molécules de tensioactif qui les entouraient ainsi que des résidus réactionnels (monomère(s) n'ayant pas réagi, réactifs en excès), peuvent alors être récupérés et être aisément dispersés, éventuellement sous ultra-sons, dans un solvant organique tel que la *N*-méthylpyrrolidone ou le *N,N*-diméthylformamide.

On obtient ainsi des suspensions stables de nano-objets en carbone, que l'on peut diluer par de nombreux solvants organiques (méthanol, éthanol, acétate d'éthyle, tétrahydrofurane, dichlorométhane, chloroforme, toluène, etc) et conserver, ainsi diluées ou non, plusieurs mois sans risque de précipitation.

De plus, les nano-objets fonctionnalisés présents dans ces suspensions peuvent aisément être séparés du solvant organique dans lequel ils se trouvent, par exemple par filtration, et être soumis à des manipulations ultérieures sans risque d'altération de leur structure et/ou de leurs propriétés.

Ainsi, il est notamment possible de compléter la fonctionnalisation de ces nano-objets par formation, sur la couche de polymère réticulé qui les entoure, d'une ou plusieurs couches de polymère(s) réticulé(s) additionnelles, auquel cas on répète l'enchaînement des étapes a) à f) de ce procédé autant que fois que le nombre de couches de polymère(s) réticulé(s) additionnelles que l'on souhaite former.

Le procédé de l'invention présente, en plus des avantages précédemment mentionnés, ceux :
- d'offrir un large choix de fonctionnalisations puisque la fonctionnalisation peut être aussi bien réalisée sous la forme d'une couche ou de plusieurs couches de polymère(s), ces couches pouvant être aussi bien identiques entre elles que différentes les unes des autres, de nature exclusivement organique que de nature organo-inorganique, et pouvant aussi bien comprendre un homopolymère qu'un copolymère ;
- d'être applicable à toutes sortes de nano-objets en carbone : nanotubes monoparois du type CoMoCAT™ tels que commercialisés par la société Sigma-Aldrich sous les références SG 65 et SG 76, nanotubes produits par ablation laser, nanotubes mono- ou multiparois tels que commercialisés par la société Nanocyl, nanotubes de carbone monoparois tels que commercialisés par la société Carbon Solutions, Inc., nanofeuillets de graphène telles que commercialisés par la société Nanointegris sous la dénomination Puresheets™ Mono ou Quattro, etc ; et
- de ne mettre en oeuvre que des opérations que l'on peut réaliser en milieu aqueux et dans des conditions douces, notamment en termes de température, puisque ces opérations peuvent être réalisées soit à la température ambiante soit à des températures peu éloignées de la température ambiante.

Est également décrite une composition qui comprend des nano-objets en carbone en suspension dans un solvant organique et dans laquelle les nano-objets de carbone sont entourés d'au moins une couche d'un polymère réticulé.

Comme précédemment mentionné, ces nano-objets sont typiquement des nanotubes de carbone ou du graphène.

Est, en outre, décrite l'utilisation de cette composition dans la fabrication de matériaux composites, de matériaux destinés au photovoltaïque, de dispositifs de détection du type capteurs/senseurs ou biocapteurs/biosenseurs, de systèmes de photocatalyse, de systèmes de vectorisation ciblée de composés d'intérêt thérapeutique ou diagnostique ou d'agents de contraste pour l'imagerie médicale.

D'autres caractéristiques et avantages de l'invention apparaîtront mieux à la lecture du complément de description qui suit, qui se rapporte à des exemples de mise en oeuvre du procédé de l'invention.

Bien entendu, ces exemples ne sont donnés qu'à titre d'illustration de l'objet de l'invention et ne constituent en aucun cas une limitation de cet objet.

### BRÈVE DESCRIPTION DES DESSINS

La figure 1, déjà commentée, illustre schématiquement, vu en coupe transversale, un nano-objet en carbone à section droite circulaire, par exemple un nanotube de carbone, après :
   - inclusion de ce nano-objet dans des molécules d'un tensioactif (partie A de la figure 1) ;
   - pénétration d'une solution comprenant un ou plusieurs monomères dans un solvant organique jusqu'à l'interface entre le nano-objet et les molécules de tensioactif (partie B de la figure 1) ; et après
   - élimination du solvant organique, déprotection des groupes chimiques polymérisables du ou des monomères si ces groupes sont protégés, et polymérisation/réticulation du ou des monomères (partie C de la figure 1).
La figure 2 représente un premier exemple d'un monomère apte à être utilisé dans le procédé de l'invention, qui correspond à un dérivé de la 5,10,15,20-tétra-(4-carboxyphényl)porphyrine base libre, qui comporte quatre groupes thiols en tant que groupes chimiques polymérisables et dans lequel chaque groupe thiol est relié à l'un des groupes acides carboxyliques de la porphyrine par l'intermédiaire d'un groupe espaceur -NH-(CH₂)₂-.
La figure 3 représente un deuxième exemple de monomères aptes à être utilisés dans le procédé de l'invention, qui correspondent à des dérivés de 5,10,15,20-tétra(4-carboxyphényl)porphyrines métallées (M représentant un atome d'un métal, par exemple de Zn, Cu, Co, Ni, Au, Fe, Pt, etc), qui comportent quatre groupes thiols en tant que groupes chimiques polymérisables et dans lesquels chaque groupe thiol est relié à l'un des groupes acides carboxyliques de la porphyrine par l'intermédiaire d'un groupe espaceur-NH-(CH₂)₂-.

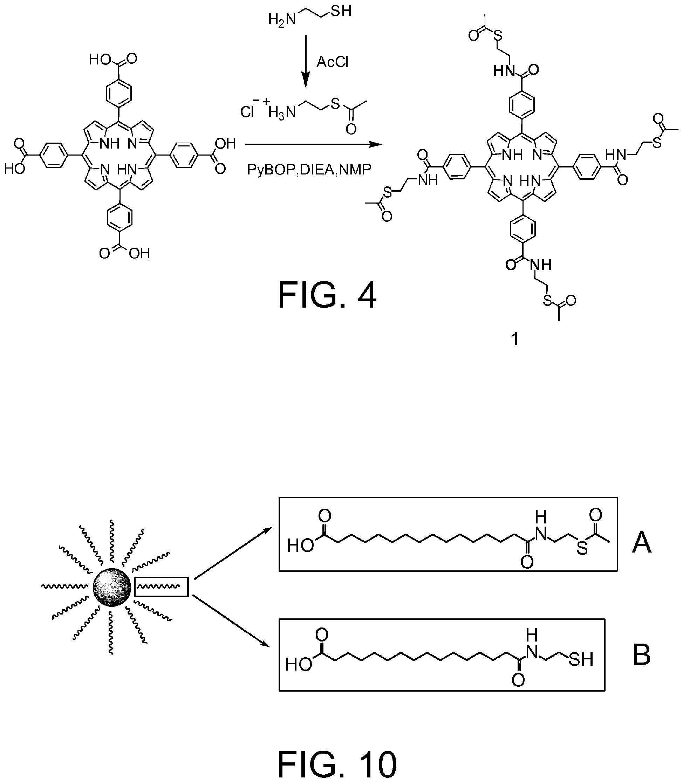

La figure 4 illustre un schéma réactionnel permettant de synthétiser le monomère de la figure 2, sous une forme dans laquelle chacun de ses groupes thiols est protégé par un groupe acétyle, à partir de la 5,10,15,20-tétra(4-carboxyphényl)-porphyrine base libre.

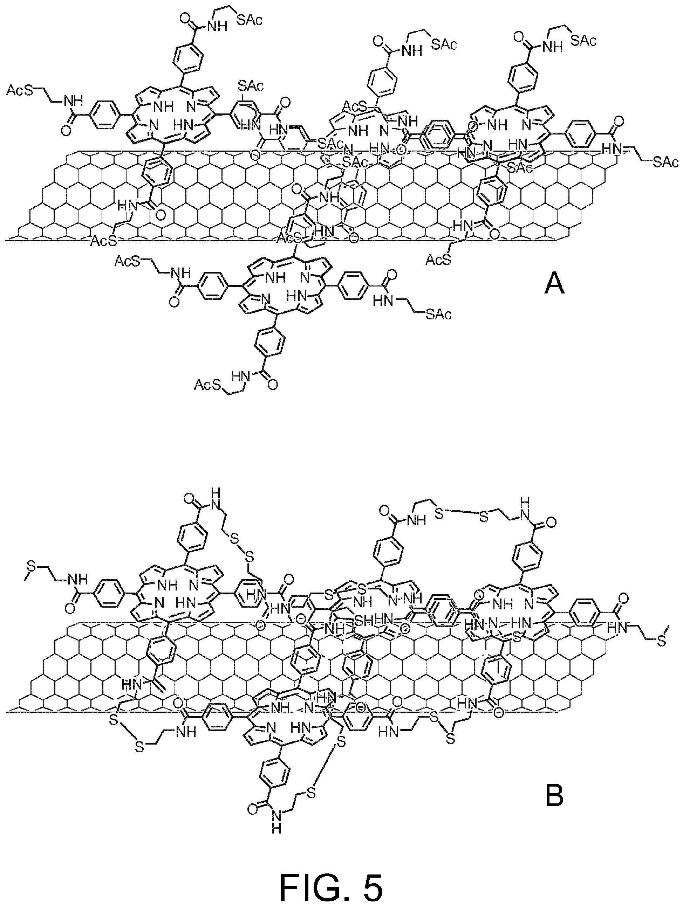

La figure 5 représente schématiquement un nanotube de carbone monoparoi tel qu'obtenu :
   - après pénétration, jusqu'à l'interface entre ce nanotube et les molécules de tensioactif qui l'entourent, d'une solution organique comprenant le monomère de la figure 2, dont chacun des groupes thiols est protégé par un groupe acétyle (partie A de la figure 5) ; et
   - après déprotection des groupes thiols de ce monomère, polymérisation/réticulation dudit monomère et élimination de la couche de molécules de tensioactif (partie B de la figure 5).

   Il est à noter que, sur la partie A de la figure 5, les molécules de tensioactif, pourtant présentes, n'ont volontairement pas été représentées pour des raisons de lisibilité.

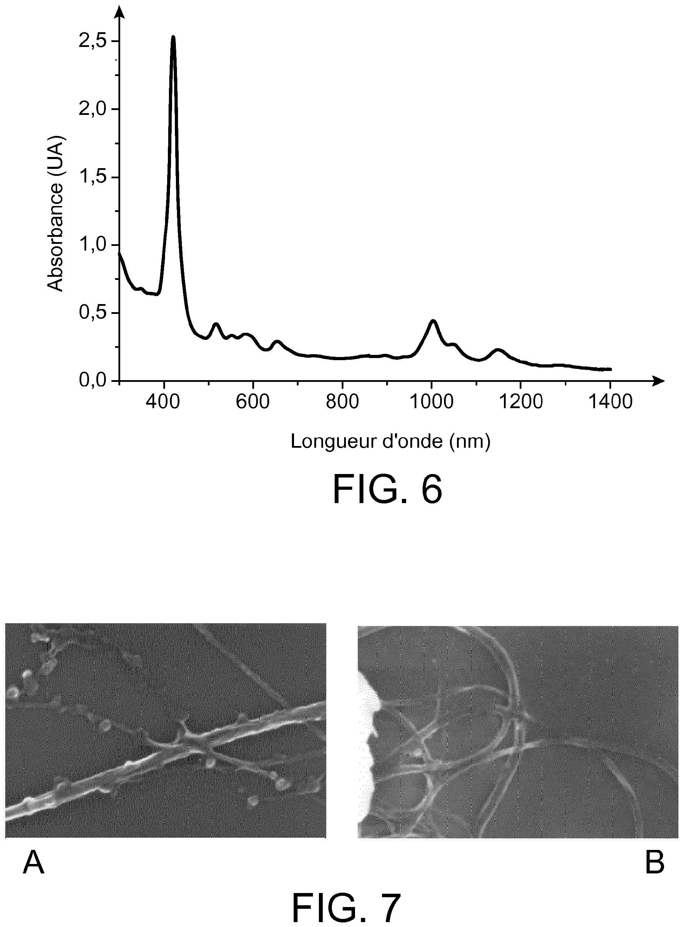

La figure 6 représente le spectre d'absorption UV-Visible-NIR (300-1400 nm) de nanotubes de carbone monoparois de type CoMoCAT™ SG 65 tels qu'illustrés sur la figure 5B, en suspension dans de la *N*-méthyl-2-pyrrolidone.
La figure 7 correspond à des images, prises au microscope électronique à balayage (MEB), de nanotubes de carbone monoparois, la partie A de cette figure correspondant à des nanotubes de carbone tels qu'illustrés sur la partie B de la figure 5, et la partie B de la figure 7 correspondant à des nanotubes de carbone n'ayant pas été fonctionnalisés par le procédé de l'invention.

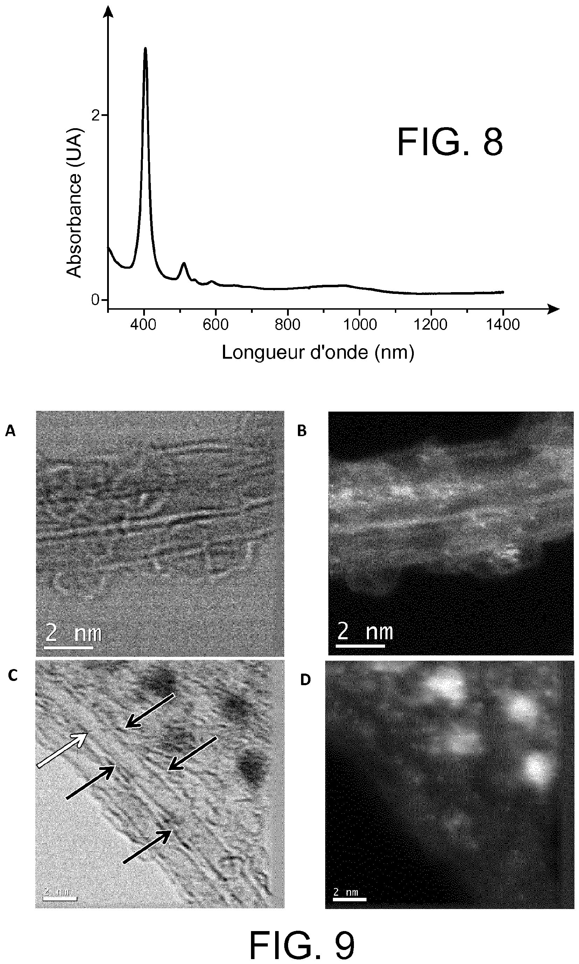

La figure 8 représente le spectre d'absorption UV-Visible-NIR (300-1400 nm) de nanotubes de carbone monoparois synthétisés par ablation laser que l'on a fonctionnalisés par une couche d'un polymère organométallique issu de la polymérisation/réticulation d'un monomère tel que représenté sur la figure 3, dans lequel M représente un atome de zinc, les nanotubes de carbone étant en suspension dans de la *N*-méthyl-2-pyrrolidone.
La figure 9 correspond à des images, prises au microscope électronique à transmission (TEM) à haute résolution, des nanotubes de carbone synthétisés par ablation laser que l'on a fonctionnalisés par une couche d'un polymère issu de la polymérisation/ réticulation d'un monomère tel que représenté sur la figure 3, dans lequel M représente un atome de platine, les parties A et C de cette figure correspondant à une observation de ces nanotubes en mode champ clair (ou mode BF de « *Bright-Field* ») et les parties B et D correspondant à une observation de ces mêmes nanotubes en mode champ sombre annulaire à grand angle (ou mode HAADF de « *High-Angle Annular Dark-Field* »)*.*
La figure 10 représente un troisième exemple de monomères aptes à être utilisés dans le procédé de l'invention, qui correspondent à des particules d'un métal, d'un semi-conducteur, d'un alliage à propriétés semi-conductrices ou d'un oxyde métallique, stabilisées par des molécules d'un ligand dérivé d'acide hexadécanedioïque, qui comporte un groupe thiol en tant que groupe chimique polymérisable et dans lequel ce groupe thiol est relié à l'un des groupes acides carboxyliques terminaux du ligand par l'intermédiaire d'un groupe espaceur -NH-(CH₂)₂-; la partie A de la figure 10 montre la formule à laquelle répond le ligand lorsque son groupe thiol est protégé par un groupe acétyle, tandis que la partie B de cette figure montre la formule à laquelle répond ce même ligand lorsque son groupe thiol n'est pas protégé.

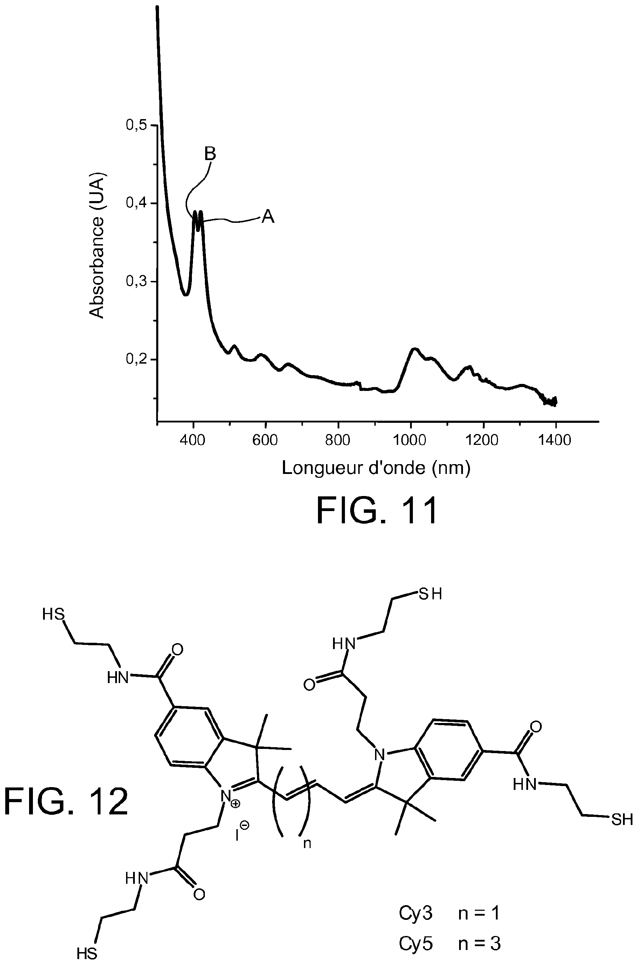

La figure 11 représente le spectre d'absorption UV-Visible-NIR (300-1400 nm) de nanotubes de carbone monoparois de type CoMoCAT™ SG 65 que l'on a fonctionnalisés par deux couches de polymères différentes, dont l'une est une couche d'un polymère organique issu de la polymérisation/réticulation du monomère de la figure 2, tandis que l'autre est une couche d'un polymère organométallique issu de la polymérisation/réticulation d'un monomère tel que représenté sur la figure 3, dans lequel M représente un atome de platine, les nanotubes de carbone étant en suspension dans de la *N*-méthyl-2-pyrrolidone.
La figure 12 représente un quatrième exemple de monomères aptes à être utilisés dans le procédé de l'invention, qui correspondent à des dérivés tétracarboxylés de la cyanine 3 (n = 1) et de la cyanine 5 (n = 3), qui comportent quatre groupes thiols en tant que groupes polymérisables et dans lesquels chaque groupe thiol est lié à un groupe acide carboxylique par l'intermédiaire d'un groupe espaceur -NH-(CH₂)₂-.

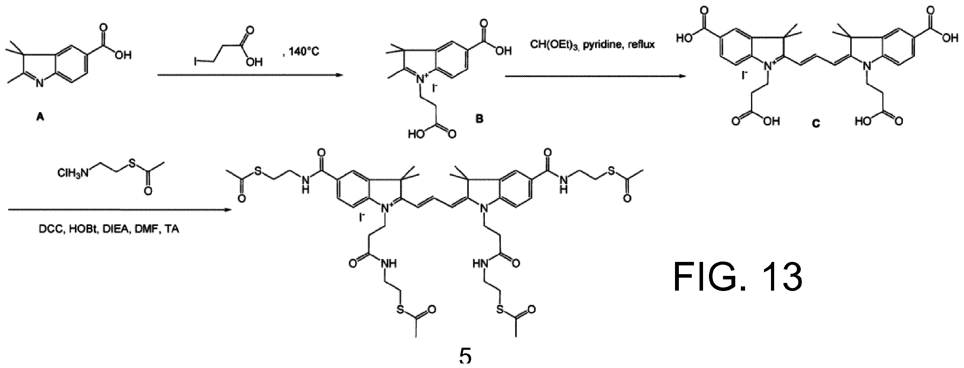

La figure 13 illustre un schéma réactionnel permettant de synthétiser le monomère tel que représenté sur la figure 12 dans lequel n = 1, sous une forme dans laquelle chacun de ses groupes thiols est protégé par un groupe acétyle, à partir de la 5-carboxyl-2,3,3-triméthyl-3*H*-indolénine.

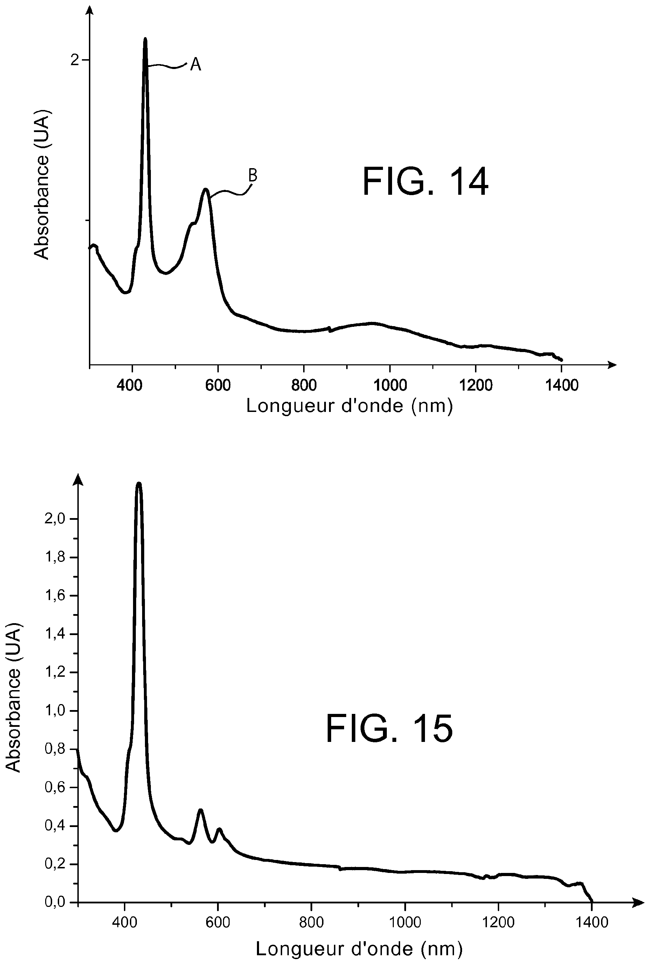

La figure 14 représente le spectre d'absorption UV-Visible-NIR (300-1400 nm) de nanotubes de carbone monoparois que l'on a fonctionnalisés par une couche d'un polymère organométallique issu de la polymérisation/réticulation de deux monomères différents, dont l'un est un monomère tel que représenté sur la figure 3, dans lequel M représente un atome de zinc, tandis que l'autre est un monomère dérivé de la cyanine 3 tel que représenté sur la figure 12, les nanotubes de carbone étant en suspension dans de la *N*-méthyl-2-pyrrolidone à 0,1% d'acide trifluoroacétique.
La figure 15 représente le spectre d'absorption UV-Visible-NIR (300-1400 nm) de nano-feuillets de graphène que l'on a fonctionnalisées par une couche d'un polymère organométallique issu de la polymérisation/réticulation d'un monomère tel que représenté sur la figure 3, dans lequel M représente un atome de zinc, les nanofeuillets de graphène étant en suspension dans de la *N*-méthyl-2-pyrrolidone.

### EXPOSÉ DÉTAILLÉ DE MODES DE MISE EN OEUVRE PARTICULIERS

### Remarque liminaire :

Dans les exemples qui suivent, toutes les sonications ont été réalisées en utilisant un bain à ultrasons électronique Elma™, modèle T490DH, d'une fréquence de 40 kHz, et délivrant à pleine puissance 130 watts avec des pics allant jusqu'à 260 watts. La puissance de sonication est modifiable d'une échelle arbitraire allant de 20 à 140%. Aussi, dans ce qui suit, une sonication douce correspond à l'utilisation d'un niveau de puissance allant de 20 à 40%, une sonication modérée correspond à l'utilisation d'un niveau de puissance allant de 60 à 80% tandis qu'une sonication forte correspond à l'utilisation d'un niveau de puissance allant de 100 à 140%.

### Exemple 1 : Fonctionnalisation de nanotubes de carbone monoparois par une couche d'un polymère organique

Dans cet exemple, on fonctionnalise des NTCs monoparois par une couche d'un polymère organique qui est issu de la polymérisation/réticulation du monomère de la figure 2, dénommé ci-après monomère **1.**

Pour ce faire, on prépare, d'une part, une suspension aqueuse de NTCs en dispersant 0,060 mg de NTCs CoMoCAT™ SG 65 (Sigma-Aldrich) dans 4 mL d'une solution aqueuse de cholate de sodium à 2% en masse et, d'autre part, une solution comprenant 1 mg du monomère **1,** sous une forme dans laquelle chacun de ses groupes thiols est protégé par un groupe acétyle (-CO-CH₃), dans 600 µL de dichlorométhane (DCM).

Le monomère **1,** avec ses groupes thiols protégés, a été préalablement obtenu à partir de la 5,10,15,20-tétra(4-carboxyphényl)porphyrine base-libre, en faisant réagir, comme montré sur la figure 4, cette porphyrine avec du chlorhydrate de S-acétylcystéamine en présence d'hexafluorophosphate de benzotriazol-1-yl-oxy-tripyrrolidinophosphonium (PyBOP) et de *N*-éthyl-*N*,*N*-diisopropylamine (DIEA) dans la *N*-méthyl-2-pyrrolidone (NMP), dans des conditions standard. Le chlorhydrate de S-acétylcystéamine a, lui-même, été préalablement obtenu par réaction entre la cystéamine et le chlorure d'acétyle.

Pour fonctionnaliser les NTCs, on ajoute, dans un réacteur, la solution organique de monomère **1** à la suspension aqueuse de NTCs.

On soumet alors les deux phases présentes dans le réacteur à une sonication forte, d'une durée de 30 minutes, pour obtenir le mélange de ces phases et la pénétration de la solution organique de monomère **1** jusqu'à l'interface entre les NTCs et les molécules de cholate de sodium qui les entourent.

Puis, on effectue une sonication douce, d'une durée d'une heure, avec un chauffage à 40°C, pour obtenir l'évaporation du DCM sans désorganiser les molécules de cholate de sodium qui entourent les NTCs.

On obtient alors une suspension aqueuse de NTCs dans laquelle ces NTCs sont entourés d'une couche de molécules de monomère **1** - comme illustré schématiquement sur la partie A de la figure 5 -, cette couche de molécules étant, elle-même, entourée de molécules de cholate de sodium.

Une solution aqueuse d'hydroxylamine 17 M (400 équivalents par rapport au monomère **1**) est alors ajoutée dans le réacteur et le contenu du réacteur est agité pendant 1 heure, à température ambiante, pour déprotéger les groupes thiols du monomère **1.**

Puis, de la triéthylamine (400 équivalents par rapport au monomère **1**) est ajoutée au réacteur et le contenu du réacteur est agité pendant 14 heures, sous atmosphère d'oxygène, pour permettre au monomère **1** de polymériser et de réticuler.

Après quoi, le contenu du réacteur est filtré sur une membrane de polytétrafluoroéthylène (Téflon™), ayant des pores de 0,2 *µ*m de diamètre, et le résidu retenu sur cette membrane est rincé successivement avec de larges volumes d'eau, de méthanol, d'acétone, de NMP, de tétrahydrofurane (THF) et de DCM, pour éliminer les molécules du monomère **1** n'ayant pas réagi, les réactifs en excès ainsi que les molécules de cholate de sodium entourant les NTCs.

Puis, le résidu est dispersé dans 4 mL de NMP anhydre, sous une sonication modérée, pendant 10 minutes.

On obtient ainsi une suspension de NTCs dans de la NMP, dans laquelle les NTCs sont fonctionnalisés par une couche d'un polymère résultant de la réaction des molécules de monomère **1** entre elles et formant enveloppe autour des NTCs, comme illustré schématiquement sur la partie B de la figure 5.

Le spectre d'absorption UV-Visible-NIR (300-1400 nm) des NTCs fonctionnalisés ainsi obtenus, en suspension dans la NMP (après dilution de cette suspension pour éviter une saturation du signal émis par les motifs répétitifs issus du monomère **1**), est illustré sur la figure 6. Ce spectre présente un pic à 420 nm qui confirme la présence, autour des NTCs, de ladite couche de polymère.

Par ailleurs, les images prises au MEB de la figure 7 mettent en évidence une différence entre l'aspect que présentent les NTCs ainsi fonctionnalisés (partie A de la figure 7) et celui qu'ils présentaient avant fonctionnalisation (partie B de la figure 7).

### Exemple 2 : Fonctionnalisation de nanotubes de carbone monoparois par une couche d'un polymère organométallique

Dans cet exemple, on fonctionnalise des NTCs monoparois par une couche d'un polymère organométallique, issu de la polymérisation/réticulation d'un monomère tel qu'illustré sur la figure 3, dans lequel M représente un atome de platine, ci-après dénommé monomère **2.**

Pour ce faire, on prépare, d'une part, une suspension aqueuse de NTCs en dispersant 0,12 mg de NTCs monoparois synthétisés par ablation laser (Docteur Oliver Jost - Université de Dresde - Allemagne), de diamètre moyen de 1,3 nm, dans 6 mL d'une solution aqueuse de dodécylsulfate de sodium à 9,84 mmol/L et, d'autre part, une solution comprenant 8 mg du monomère **2,** sous une forme dans laquelle chacun de ses groupes thiols est protégé par un groupe acétyle, dans 600 µL de DCM.

Le monomère **2,** avec ses groupes thiols protégés, a été préalablement synthétisé à partir de la 5,10,15,20-tétra(4-carboxyphényl)porphyrine de platine selon un schéma réactionnel analogue à celui représenté sur la figure 4.

Pour fonctionnaliser les NTCs, on ajoute, dans un réacteur, la solution organique de monomère **2** à la suspension aqueuse de NTCs.

Puis, on suit le même protocole opératoire que celui décrit dans l'exemple 1 ci-avant, exception faite que le résidu de filtration, une fois lavé, est dissous dans 6 mL (au lieu de 4 mL) de NMP anhydre.

Le spectre d'absorption UV-Visible-NIR (300-1400 nm) des NTCs fonctionnalisés ainsi obtenus, en suspension dans la NMP (après dilution de cette suspension pour éviter une saturation du signal émis par les motifs répétitifs issus du monomère **2**), est illustré sur la figure 8.

Ce spectre présente un pic à 400 nm qui confirme la présence autour des NTCs d'un polymère résultant de la réaction de molécules du monomère **2** entre elles.

Par ailleurs, les images prises au TEM à haute résolution de la figure 9 mettent en évidence la présence, à la surface des NTCs (illustrée par la flèche blanche), de chaînes formées par des motifs répétitifs issus de ce monomère (illustrés par les flèches noires).

### Exemple 3 : Fonctionnalisation de nanotubes de carbone monoparois par une couche d'un polymère organométallique

Dans cet exemple, on fonctionnalise des NTCs monoparois par une couche d'un polymère organométallique qui est issu de la polymérisation/ réticulation d'un monomère tel qu'illustré sur la figure 10, dans lequel la particule est une particule d'oxyde ferrique Fe₃O₄, dénommé ci-après monomère **3.**

Pour ce faire, on prépare, d'une part, une suspension aqueuse de NTCs en dispersant 0,08 mg de NTCs synthétisés par ablation laser (Docteur Oliver Jost - Université de Dresde - Allemagne), dans 4 mL d'une solution de dodécylsulfate de sodium (SDS) à 9,84 mmol/L et, d'autre part, une solution comprenant 3,5 mg du monomère **3,** sous une forme dans laquelle le groupe thiol de chacune de ses molécules de ligand est protégé par un groupe acétyle, dans 400 µL de toluène.

Le monomère **3,** avec ses groupes thiols protégés, a été préalablement obtenu à partir de particules d'oxyde ferrique stabilisées par des molécules d'acide oléique (Sigma-Aldrich, référence 700312) en remplaçant ces molécules d'acide oléique par des molécules du ligand montré sur la partie A de la figure 10, par la méthode d'échange de ligands décrite par Lattuada et Hatton (Langmuir 2007, 23, 2158-2168, référence **[3]**).

Pour fonctionnaliser les NTCs, on ajoute, dans un réacteur, la solution organique de monomère **3** à la suspension aqueuse de NTCs.

On soumet alors les deux phases présentes dans le réacteur à une sonication forte, d'une durée de 30 minutes, pour obtenir le mélange de ces phases et la pénétration de la solution organique de monomère **3** jusqu'à l'interface entre les NTCs et les molécules de SDS qui les entourent.

Puis, on effectue une sonication douce, d'une durée d'une heure, avec un chauffage à 40°C, mais en plaçant le réacteur sous vide, pour obtenir l'évaporation du toluène.

On suit ensuite le même protocole opératoire que celui décrit dans l'exemple 1 ci-avant, exception faite que le résidu de filtration, une fois lavé, est dispersé dans 4 mL d'un mélange NMP/toluène anhydre (au lieu de NMP anhydre seule).

### Exemple 4 : Fonctionnalisation de nanotubes de carbone monoparois par deux couches de polymères de nature différente

Dans cet exemple, on fonctionnalise des NTCs monoparois, déjà fonctionnalisés par une première couche d'un polymère issu de la polymérisation/ réticulation du monomère **1,** par une deuxième couche d'un polymère organométallique qui est, lui, issu de la polymérisation/réticulation du monomère **2.**

Pour ce faire, on ajoute, dans un réacteur, 1 mL d'une suspension comprenant 0,06 mg de NTCs fonctionnalisés, tels qu'obtenus dans l'exemple 1 ci-avant, dans de la NMP, à 10 mL d'une solution aqueuse de cholate de sodium à 2%.

Puis, on ajoute 500 µL d'une solution comprenant 50 µg du monomère **2,** sous une forme dans laquelle chacun de ses groupes thiols est protégé par un groupe acétyle, dans du DCM, de manière à obtenir 2 équivalents de monomère **2** par rapport au polymère déjà présent à la surface des NTCs.

On suit ensuite le même protocole opératoire que celui décrit dans l'exemple 1 ci-avant, exception faite que le résidu de filtration, une fois lavé, est dissous dans 2 mL (au lieu de 4 mL) de NMP anhydre.

Le spectre d'absorption UV-Visible-NIR (300-1400 nm) des NTCs doublement fonctionnalisés ainsi obtenus, en suspension dans la NMP (après dilution de cette suspension pour éviter une saturation des signaux émis par les motifs répétitifs issus des monomères **1** et **2**), est illustré sur la figure 11.

Ce spectre montre la présence de deux pics : un premier pic à 420 nm, noté A, qui confirme la présence, autour des NTCs, d'une première couche d'un polymère résultant de la polymérisation/réticulation du monomère **1,** et un deuxième pic à 400 nm, noté B, qui confirme la présence, autour des NTCs, d'une deuxième couche d'un polymère résultant de la réaction de molécules du monomère **2** entre elles.

### Exemple 5 : Fonctionnalisation de nanotubes de carbone monoparois par une couche d'un copolymère organométallique

Dans cet exemple, on fonctionnalise des NTCs monoparois par un copolymère organométallique qui est issu de la copolymérisation/réticulation d'un monomère tel qu'illustré sur la figure 3, dans lequel M représente un atome de zinc, ci-après monomère **4,** et d'un monomère dérivé de la cyanine 3 tel qu'illustré sur la figure 12, ci-après monomère **5.**

Pour ce faire, on prépare, d'une part, une suspension aqueuse comprenant 0,20 mg de NTCs synthétisés par ablation laser (Docteur Oliver Jost - Université de Dresde - Allemagne), dans 10 mL d'une solution aqueuse de SDS à 9,84 mmol/L et, d'autre part, une solution comprenant, d'une part, 5 mg du monomère **4,** sous une forme dans laquelle chacun de ses groupes thiols est protégé par un groupe acétyle, et, d'autre part, 5,7 mg du monomère **5,** également sous une forme dans laquelle chacun de ses groupes thiols est protégé par un groupe acétyle, dans 600 µL de DCM.

Le monomère **4,** aux groupes thiols protégés, a été préalablement obtenu à partir du monomère tel qu'illustré sur la figure 4, en faisant réagir ce monomère avec du diacétate de zinc (Zn(OAc)₂) dans un milieu composé de DCM et de méthanol, selon une procédure analogue à celle décrite par Wagner et al. (J. Org. Chem. 1995, 60(16), 5266-5273, référence **[4]**) à ceci près que du DCM a été utilisé à la place du chloroforme comme solvant.

Le monomère **5,** aux groupes thiols protégés, a été préalablement obtenu à partir de la 5-carboxyl-2,3,3-triméthyl-3*H*-indolénine, en suivant le schéma réactionnel montré sur la figure 13, la 5-carboxyl-2,3,3-triméthyl-3*H*-indolénine, notée **A** sur cette figure, ayant été elle-même préalablement obtenue en suivant la procédure décrite par Terpetschnig et al. (Analytical Biochemistry 1994, 217, 197-204, référence **[5]**).

Pour obtenir le monomère **5,** le composé **A** (200 mg, 1 mmol) et l'acide 3-iodopropanoïque (1 g, 5 mmol) ont été introduits dans un tube scellé chauffé à 140°C pendant 4 heures. Le mélange réactionnel contenant le composé, noté **B** sur la figure 13, a été refroidi à 100°C puis de la pyridine anhydre (8 mL) a été ajoutée. La suspension a été transférée dans un ballon, de l'orthoformiate d'éthyle (40 *µ*L, 242 *µ*mol) a été ajouté et la suspension a été chauffée à reflux. Deux additions successives d'orthoformiate d'éthyle (40 *µ*L, 242 *µ*mol) espacées de 2 heures sont effectuées ensuite. L'avancement de la réaction est contrôlé par absorption UV-Visible avec l'apparition d'une bande à 550 nm correspondant au composé qui est noté **C** sur la figure 13. La réaction est stoppée lorsque la bande à 550 nm cesse d'évoluer.

Le solvant est évaporé et le brut réactionnel est dissous dans de l'acétate d'éthyle (125 mL) et il est lavé avec une solution aqueuse à 10% en masse d'acide citrique (125 mL) puis avec une solution de NaCl saturée (125 mL). Le composé **C** est extrait de la phase organique avec une solution saturée de NaHCO₃ (125 mL). Les phases sont séparées et la solution aqueuse est lavée avec du dichlorométhane (125 mL) puis acidifiée avec de l'HCl 1 M jusqu'à obtenir un pH de 2. La suspension est extraite avec de l'acétate d'éthyle (200 mL), séchée sur Na₂SO₄ anhydre, filtrée et évaporée. Le composé **C** est obtenu sous forme d'un solide rouge.

Ce composé (200 mg, 291 *µ*mol) est dissous dans de la NMP anhydre, et du chlorhydrate de S-acétylcystéamine (270 mg, 1,74 mmol), du PyBOP (904 mg, 1,74 mmol) et de la DIEA (912 *µ*L, 5,2 mmol) sont ajoutés. Le milieu réactionnel est agité à température ambiante pendant 3 heures puis dilué avec de l'acétate d'éthyle (80 mL). La solution est lavée avec une solution aqueuse à 10% en masse d'acide citrique (80 mL) puis avec une solution de NaHCO₃ saturée (80 mL) puis avec une solution de NaCl saturée (80 mL). La phase organique est séchée sur Na₂SO₄ anhydre, filtrée et évaporée. On obtient ainsi le monomère **5.**

Pour fonctionnaliser les NTCs, on ajoute, dans un réacteur, la solution organique de monomères **4** et **5** à la suspension aqueuse de NTCs.

On soumet alors les deux phases présentes dans le réacteur à une sonication forte, d'une durée de 30 minutes, pour obtenir le mélange de ces phases et la pénétration de la solution organique de monomères **3** et **5** jusqu'à l'interface entre les NTCs et les molécules de SDS qui les entourent.

Puis, on effectue une sonication douce, d'une durée d'une heure, avec un chauffage à 40°C, pour obtenir l'évaporation du DCM sans déstabiliser les molécules de SDS qui entoure les NTCs.

Une solution aqueuse d'ammoniac à 28% (400 équivalents par rapport au monomère **4** et **5**) est alors ajoutée dans le réacteur et le contenu du réacteur est agité, pendant 1 heure à température ambiante, pour déprotéger les groupes thiols des monomères **4** et **5.** Le contenu du réacteur est ensuite agité pendant 14 heures, sous atmosphère d'oxygène, pour permettre aux monomères de copolymériser et de réticuler.

Après quoi, le contenu du réacteur est filtré sur une membrane de polytétrafluoroéthylène (Téflon™), ayant des pores de 0,2 *µ*m de diamètre, et le résidu retenu sur cette membrane est rincé successivement avec de larges quantités d'eau, de méthanol, d'acétone, de NMP, de THF et de DCM.

Puis, le résidu est dispersé dans 6 mL de NMP anhydre, sous une sonication modérée, pendant 10 minutes.

Le spectre d'absorption UV-Visible-NIR (300-1400 nm) des NTCs fonctionnalisés ainsi obtenus, en suspension dans la NMP à 0,1% de TFA (après dilution de cette suspension pour éviter une saturation des signaux émis par les motifs répétitifs issus des monomères **4** et **5**), est illustré sur la figure 13.

Ce spectre montre la présence de deux pics, notés A et B, situés respectivement à 430 nm et 570 nm, qui confirme la présence, autour des NTCs d'un copolymère résultant de la réaction de molécules des monomères **4** et **5** entre elles.

### Exemple 6: Fonctionnalisation de nanofeuillets de graphène par une couche d'un polymère organométallique

Dans cet exemple, on fonctionnalise des nanofeuillets de graphène par un polymère organométallique qui est issu de la polymérisation/réticulation du monomère **4.**

Pour ce faire, on ajoute 600 µL d'une solution comprenant 3 mg du monomère **4,** sous une forme dans laquelle chacun de ses groupes thiols est protégé par un groupe acétyle, dans du DCM à 6 mL d'une suspension aqueuse qui comprend 0,30 mg d'un mélange de nanofeuillets de graphène monocouches (27%), bicouches (48%), tricouches (20%), tétracouches et plus (5%) dans un tensioactif, et qui est commercialisée par la société Nanointegris sous la dénomination PureSheets Mono™.

Puis, on suit le même protocole opératoire que celui décrit dans l'exemple 1 ci-avant.

Le spectre d'absorption UV-Visible-NIR (300-1400 nm) des nanofeuillets de graphène fonctionnalisés ainsi obtenus, en suspension dans la MNP, est illustré sur la figure 14.

Ce spectre montre la présence d'un pic à 420 nm qui confirme la présence, autour des nanofeuillets de graphène, d'un polymère résultant de la réaction de molécules du monomère **4** entre elles.

### RÉFÉRENCES CITÉES

**[1]** Demande de brevet US 2011/0076497
**[2]** Sriya Das et al., ACS Applied Materials & Interfaces 2011, 3, 1844-1851
**[3]** Lattuada et Hatton, Langmuir 2007, 23, 2158-2168
**[4]** Wagner et al., J. Org. Chem. 1995, 60(16), 5266-5273
**[5]** Terpetschnig et al., Analytical Biochemistry 1994, 217, 197-204

## Revendications

1. Procédé de fonctionnalisation de nano-objets en carbone par formation d'au moins une couche d'un polymère réticulé autour de ces nano-objets, qui comprend au moins les étapes suivantes :
a) la dispersion des nano-objets dans une solution aqueuse d'un tensioactif pour obtenir une suspension dans laquelle chaque nano-objet est entouré de molécules de tensioactif, la partie hydrophobe de ces molécules étant orientée vers le nano-objet et la partie hydrophile desdites molécules étant en contact avec l'eau de la suspension ;
b) le mélange de la suspension ainsi obtenue avec une solution comprenant un ou plusieurs monomères organiques et/ou organo-inorganiques dans un solvant organique non miscible à l'eau, le ou les monomères ayant un coefficient de partage dichlorométhane/eau déionisée au moins égal à 5 à une température de 25°C et comportant au moins trois groupes chimiques polymérisables choisis parmi les groupes thiols et sélénols, éventuellement protégés par un groupe protecteur, ce mélange étant réalisé sous agitation, moyennant quoi la solution de monomère(s) est amenée à l'interface entre les nano-objets et les molécules de tensioactif qui entourent ces nano-objets ;
c) l'élimination du solvant organique du mélange ;
d) la polymérisation et la réticulation du ou des monomères à l'interface entre les nano-objets et les molécules de tensioactif qui entourent ces nano-objets, après déprotection des groupes chimiques polymérisables si ceux-ci sont protégés, pour obtenir la formation d'une couche de polymère réticulé autour des nano-objets, cette couche étant elle-même entourée par les molécules de tensioactif ;
e) l'élimination des molécules de tensio-actif qui entourent la couche de polymère réticulé ; et
f) la récupération des nano-objets ainsi fonctionnalisés.

2. Procédé selon la revendication 1, dans lequel les nano-objets sont des nanotubes de carbone ou des nanofeuillets de graphène.

3. Procédé selon la revendication 1 ou la revendication 2, dans lequel le ou les monomères sont choisis parmi :
- les composés comprenant un chromophore ;
- les complexes d'un métal de transition, dans lesquels ce métal est coordonné à plusieurs molécules d'un même ligand organique ou à plusieurs molécules correspondant à des ligands organiques différents ;
- les complexes d'une terre rare, dans lesquels cette terre rare est coordonnée à plusieurs molécules d'un même ligand organique ou à plusieurs molécules correspondant à des ligands organiques différents ; et
- des nanoparticules inorganiques stabilisées par un ligand organique.

4. Procédé selon la revendication 3, dans lequel le ou les monomères sont choisis parmi les composés comprenant un groupe azobenzène, anthraquinone, indigotine, triarylméthane, acridine, xanthène, β-carotène, quinoline, chlorine, porphyrine, phtalocyanine, naphtalocyanine, fluorescéine, rhodamine, bore-dipyrométhène, coumarine ou cyanine.

5. Procédé selon l'une quelconque des revendications précédentes, dans lequel le ou les monomères comportent au moins quatre groupes chimiques polymérisables.

6. Procédé selon l'une quelconque des revendications précédentes, dans lequel les groupes chimiques polymérisables sont situés à l'extrémité de groupes espaceurs que comportent le ou les monomères.

7. Procédé selon l'une quelconque des revendications précédentes, dans lequel, à l'étape b), l'agitation du mélange est réalisée par une sonication.

8. Procédé selon l'une quelconque des revendications précédentes, dans lequel l'étape c) comprend une élévation de la température du mélange conjointement avec une sonication de ce mélange.

9. Procédé selon la revendication 8, dans lequel le mélange est porté à une température allant de 40 à 50°C.

10. Procédé selon la revendication 1, dans lequel le ou les monomères utilisés à l'étape b) comportent des groupes thiols ou sélénols protégés par un groupe acétyle, et l'étape d) comprend le traitement des nano-objets obtenus à l'issue de l'étape c) :
- par un agent de désacétylation que l'on utilise en excès par rapport aux groupes thiols ou sélénols, puis
- par une base que l'on utilise également en excès par rapport aux groupes thiols ou sélénols, sous une atmosphère oxydante.

11. Procédé selon la revendication 10, dans lequel le traitement des nano-objets est réalisé à température ambiante.

12. Procédé selon l'une quelconque des revendications précédentes, dans lequel l'étape e) comprend une série de rinçage des nano-objets obtenus à l'issue de l'étape d), ces rinçages étant successivement réalisés avec de l'eau et des solvants organiques.

13. Procédé selon l'une quelconque des revendications précédentes, qui comprend de plus la dispersion des nano-objets obtenus à l'issue de l'étape e) dans un solvant organique, de préférence la *N*-méthylpyrrolidone ou le *N,N*-diméthylformamide.

14. Procédé selon l'une quelconque des revendications précédentes, dans lequel l'enchaînement des étapes a) à f) est répété une ou plusieurs fois.

## Patentansprüche

1. Verfahren zur Funktionalisierung von Kohlenstoff-Nanoobjekten durch Bildung zumindest einer Schicht aus einem um diese Nanoobjekte herum vernetzten Polymer, das zumindest die nachfolgenden Schritte umfasst:
a) Dispergieren der Nanoobjekte in einer wässrigen Lösung eines Tensids, um eine Suspension zu erhalten, in der jedes Nanoobjekt von Tensidmolekülen umgeben ist, wobei der hydrophobe Teil dieser Moleküle zu dem Nanoobjekt hin ausgerichtet ist und der hydrophile Teil der Moleküle mit dem Wasser der Suspension in Kontakt ist;
b) Vermischen der so erhaltenen Suspension mit einer Lösung, die ein oder mehrere organische und/oder organisch-anorganische Monomere in einem mit Wasser nicht mischbaren organischen Lösungsmittel enthält, wobei das bzw. die Monomer(e) einen Verteilungskoeffizienten von Dichlormethan/entionisiertem Wasser von mindestens gleich 5 bei einer Temperatur von 25 °C aufweisen und zumindest drei polymerisierbare chemische Gruppen, ausgewählt aus Thiol- und Selenolgruppen, gegebenenfalls geschützt durch eine Schutzgruppe, enthalten, wobei dieses Vermischen unter Rühren durchgeführt wird, wodurch die Lösung aus Monomer(en) an die Grenzfläche zwischen den Nanoobjekten und den diese Nanoobjekte umgebenden Tensidmolekülen gebracht wird;
c) Entfernen des organischen Lösungsmittels aus dem Gemisch;
d) Polymerisieren und Vernetzen des bzw. der Monomere an der Grenzfläche zwischen den Nanoobjekten und den diese Nanoobjekte umgebenden Tensidmolekülen nach Entschützen der polymerisierbaren chemischen Gruppen, falls diese geschützt sind, um die Bildung einer Schicht aus um die Nanoobjekte herum vernetztem Polymer zu erhalten, wobei diese Schicht selbst von den Tensidmolekülen umgeben ist; und
e) Entfernen der Tensidmoleküle, die die Schicht aus vernetztem Polymer umgeben; und
f) Gewinnen der so funktionalisierten Nanoobjekte.

2. Verfahren nach Anspruch 1, wobei die Nanoobjekte Kohlenstoff-Nanoröhrchen oder Graphen-Nanoplättchen sind.

3. Verfahren nach Anspruch 1 oder Anspruch 2, wobei das bzw. die Monomer(e) ausgewählt sind aus:
- Verbindungen, die ein Chromophor enthalten;
- Komplexen eines Übergangsmetalls, in denen dieses Metall an mehrere Moleküle eines gleichen organischen Liganden oder an mehrere Moleküle, die verschiedenen organischen Liganden entsprechen, koordiniert ist;
- Komplexen einer seltenen Erde, in denen diese seltene Erde mit mehreren Molekülen eines gleichen organischen Liganden oder mit mehreren Molekülen, die verschiedenen organischen Liganden entsprechen, koordiniert ist; und
- anorganischen Nanopartikeln, die mit einem organischen Liganden stabilisiert sind.

4. Verfahren nach Anspruch 3, wobei das bzw. die Monomer(e) ausgewählt sind aus Verbindungen, enthaltend eine Azobenzol-, Anthrachinon-, Indigotin-, Triarylmethan-, Acridin-, Xanthen-, β-Carotin-, Chinolin-, Chlorin-, Porphyrin-, Phthalocyanin-, Naphthalocyanin-, Fluorescein-, Rhodamin-, Bor-Dipyromethen-, Cumarin oder Cyaningruppe.

5. Verfahren nach einem der vorangehenden Ansprüche, wobei das bzw. die Monomer(e) zumindest vier polymerisierbare chemische Gruppen enthalten.

6. Verfahren nach einem der vorangehenden Ansprüche, wobei die polymerisierbaren chemischen Gruppen sich am Ende von Abstandshaltergruppen befinden, die das bzw. die Monomer(e) enthalten.

7. Verfahren nach einem der vorangehenden Ansprüche, wobei in Schritt b) das Rühren des Gemischs durch Ultraschallbehandlung durchgeführt wird.

8. Verfahren nach einem der vorangehenden Ansprüche, wobei Schritt c) eine Erhöhung der Temperatur des Gemischs zusammen mit einer Ultraschallbehandlung dieses Gemisches umfasst.

9. Verfahren nach Anspruch 8, wobei das Gemisch auf eine Temperatur von 40 bis 50 °C gebracht wird.

10. Verfahren nach Anspruch 1, wobei das bzw. die in Schritt b) verwendeten Monomer(e) Thiol- oder Selenolgruppen enthalten, die durch eine Acetylgruppe geschützt sind, und Schritt d) die Behandlung der am Ende von Schritt c) erhaltenen Nanoobjekte umfasst:
- mit einem Deacetylierungsmittel, das im Überschuss gegenüber den Thiol- oder Selenolgruppen verwendet wird, dann
- mit einer Base, die auch im Überschuss gegenüber den Thiol- oder Selenolgruppen verwendet wird, in einer oxidierenden Atmosphäre.

11. Verfahren nach Anspruch 10, wobei die Behandlung der Nanoobjekte bei Raumtemperatur erfolgt.

12. Verfahren nach einem der vorangehenden Ansprüche, wobei Schritt e) eine Reihe von Spülungen der Nanoobjekte umfasst, die am Ende von Schritt d) erhalten wurden, wobei diese Spülungen nacheinander mit Wasser und organischen Lösungsmitteln durchgeführt werden.

13. Verfahren nach einem der vorangehenden Ansprüche, das ferner das Dispergieren der am Ende von Schritt e) erhaltenen Nanoobjekte in einem organischen Lösungsmittel, vorzugsweise N-Methylpyrrolidon oder N,N-Dimethylformamid, umfasst.

14. Verfahren nach einem der vorangehenden Ansprüche, wobei die Abfolge der Schritte a) bis f) ein oder mehrere Male wiederholt wird.

## Claims

1. Method for functionalization of carbon nano-objects by forming at least one layer of a crosslinked polymer around these nano-objects, which comprises at least the following steps:
a) dispersing nano-objects in an aqueous solution of a surfactant to obtain a suspension in which each nano-object is surrounded with surfactant molecules, the hydrophobic portion of these molecules being oriented towards the nano-object and the hydrophilic portion of said molecules being in contact with the water of the suspension;
b) mixing the thereby obtained suspension with a solution comprising one or several organic and/or organo-inorganic monomers in an organic solvent non-miscible with water, the monomer(s) having a dichloromethane/deionized water partition coefficient at least equal to 5 at a temperature de 25°C and comprising at least three polymerizable chemical groups selected from thiol and selenol groups, optionally protected with a protective group, this mixture being made with stirring, whereby the solution of monomer(s) is brought to the interface between the nano-objects and the surfactant molecules which surround these nano-objects;
c) removing the organic solvent from the mixture;
d) polymerizing and crosslinking the monomer(s) at the interface between the nano-objects and the surfactant molecules which surround these nano-objects, after deprotection of the polymerization chemical groups if the latter are protected, to obtain the formation of a crosslinked polymer layer around the nano-objects, this layer being itself surrounded by surfactant molecules;
e) removing the surfactant molecules which surround the crosslinked polymer layer; and
f) recovering the thereby functionalized nano-objects.

2. Method according to claim 1, wherein the nano-objects are carbon nanotubes or graphene nanosheets.

3. Method according to claim 1 or claim 2, wherein the monomer(s) is (are) selected from:
- compounds comprising a chromophore;
- complexes of a transition metal, in which this metal is coordinated to several molecules of a same organic ligand or to several molecules corresponding to different organic ligands;
- complexes of a rare earth, in which this rare earth is coordinated to several molecules of a same organic ligand or to several molecules corresponding to different organic ligands; and
- inorganic nanoparticles stabilized by an organic ligand.

4. Method according to claim 3, wherein the monomer(s) is (are) selected from compounds comprising an azobenzene, anthraquinone, indigotin, triarylmethane, acridine, xanthene, β-carotene, quinoline, chlorin, porphyrin, phthalocyanin, naphthalocyanin, fluorescein, rhodamine, bore-dipyromethene, coumarin or cyanin group.

5. Method according to any of the preceding claims, wherein the monomer(s) include(s) at least four polymerisable chemical groups.

6. Method according to any of the preceding claims, wherein the polymerizable chemical groups are located at the end of spacer groups which the monomer(s) include(s).

7. Method according to any of the preceding claims, wherein, in step b), the stirring of the mixture is achieved by sonication.

8. Method according to any of the preceding claims, wherein step c) comprises a rise in the temperature of the mixture together with sonication of this mixture.

9. Method according to claim 8, wherein the mixture is brought to a temperature ranging from 40 to 50°C.

10. Method according to claim 1, wherein the monomer(s) used in step b) include(s) thiol or selenol groups protected with an acetyl group, and step d) comprises the treatment of the nano-objects obtained at the end of step c):
- with a deacetylation agent which is used in excess relatively to the thiol or selenol groups, and then
- with a base which is also used in excess relatively to the thiol or selenol groups, under an oxidizing atmosphere.

11. Method according to claim 10, wherein the treatment of the nano-objects is carried out at room temperature.

12. Method according to any of the preceding claims, wherein step e) comprises a series of rinsing of the nano-objects obtained at the end of step d), these rinses being successively carried out with water and organic solvents.

13. Method according to any of the preceding claims, which further comprises dispersion of the nano-objects obtained at the end of step e) in an organic solvent, preferably N-methylpyrrolidone or N,N-dimethylformamide.

14. Method according to any of the preceding claims, wherein the succession of steps a) to f) is repeated one or several times.
